# EUROPEAN PATENT SPECIFICATION

(11) **EP 3 187 878 B1**
(45) Date of publication and mention of the grant of the patent: **31.07.2019**
(21) Application number: 15835787.1
(22) Date of filing: 25.08.2015
(51) Int. Cl.: A61K 31/555, A61K 31/713, G01N 33/68, A61K 31/282, A61K 45/00, A61P 35/00, A61P 43/00, G01N 33/15, G01N 33/50, G01N 33/574

(54) **ANTI-CANCER AGENT SENSITIVITY-DETERMINING MARKER**
MARKER ZUR BESTIMMUNG DER EMPFINDLICHKEIT FÜR EIN ANTIKREBSMITTEL
MARQUEUR DE DÉTERMINATION DE SENSIBILITÉ À UN AGENT ANTICANCÉREUX

(30) Priority: 26.08.2014 JP 2014171729
(43) Date of publication of application: 05.07.2017
(60) Divisional application: 19170788.4
(73) Proprietor: Keio University, Tokyo 108-8345 (JP); Kabushiki Kaisha Yakult Honsha, Tokyo 105-8660 (JP)
(72) Inventor: TANIGAWARA, Yusuke, Tokyo 160-8582 (JP); HARA, Kanako, Tokyo 160-8582 (JP); TANAKA, Miki, Kawasaki-shi Kanagawa 210-0834 (JP); SUGIMOTO, Shinji, Tokyo 105-8660 (JP); TAKAHASHI, Hiroyuki, Tokyo 105-8660 (JP)
(74) Representative: Blodig, Wolfgang
(86) International application number: PCT/JP2015/073868
(87) International publication number: WO 2016/031816

(56) References cited:
- EP-A1- 2 241 334
- WO-A1-2012/127984
- WO-A1-2012/172370
- JP-A- H05 271 294
- JP-A- 2007 037 409
- US-A1- 2007 122 830
- US-A1- 2007 212 738
- US-A1- 2008 096 235
- US-B2- 7 713 525
- EVA MARTINEZ-BALIBREA ET AL: "Increased levels of copper efflux transporter ATP7B are associated with poor outcome in colorectal cancer patients receiving oxaliplatin-based chemotherapy", INTERNATIONAL JOURNAL OF CANCER, vol. 124, no. 12, 15 June 2009 (2009-06-15), pages 2905-2910, XP055447038, US ISSN: 0020-7136, DOI: 10.1002/ijc.24273
- RASMUSSEN MADS H ET AL: "High expression of microRNA-625-3p is associated with poor response to first-line oxaliplatin based treatment of metastatic colorectal cancer", MOLECULAR ONCOLOGY, ELSEVIER, AMSTERDAM, NL, vol. 7, no. 3, 28 February 2013 (2013-02-28), pages 637-646, XP028543819, ISSN: 1574-7891, DOI: 10.1016/J.MOLONC.2013.02.016
- VERMONT P DIA ET AL: "Lunasin potentiates the effect of oxaliplatin preventing outgrowth of colon cancer metastasis, binds tointegrin and suppresses FAK/ERK/NF-B signaling", CANCER LETTERS, NEW YORK, NY, US, vol. 313, no. 2, 1 September 2011 (2011-09-01), pages 167-180, XP028109315, ISSN: 0304-3835, DOI: 10.1016/J.CANLET.2011.09.002 [retrieved on 2011-09-10]
- YANLEI MA ET AL: "Searching for serum tumor markers for colorectal cancer using a 2-D DIGE approach", ELECTROPHORESIS : LIQUID PHASE SEPARATION TECHNIQUES : MICROFLUIDICS, NANOANALYSIS, PROTEOMICS, vol. 30, no. 15, 1 August 2009 (2009-08-01), pages 2591-2599, XP055447390, DE ISSN: 0173-0835, DOI: 10.1002/elps.200900082
- HEINER SCHAFER ET AL: "Cytoprotection "gone astray'': Nrf2 and its role in cancer", ONCOTARGETS AND THERAPY, 30 March 2014 (2014-03-30), page 1497, XP055447127, GB ISSN: 1178-6930, DOI: 10.2147/OTT.S36624
- PATEL NISH ET AL.: 'Rescue of paclitaxel sensitivity by repression of Prohibitinl in drug-resistant cancer cells' PROC NATL ACAD SCI USA vol. 107, no. 6, 09 February 2010, pages 2503 - 2508, XP055411222 DOI: 10.1073/PNAS.0910649107
- SAYO SUZUKI ET AL.: 'Search for new biomarkers to predict Oxaliplatin sensitivity by SELDI-TOF MS proteomic screening' ABSTRACTS OF ANNUAL MEETING OF PHARMACEUTICAL SOCIETY OF JAPAN vol. 128 TH, no. 3, 2008, page 110, XP009500489
- AKITO NISHIMUTA ET AL.: 'Metabolome Kaiseki ni yoru Hito Daichogan Saibo no Oxaliplatin Kanjusei Biomarker no Tansaku' JAPANESE JOURNAL OF THERAPEUTIC DRUG MONITORING vol. 28, no. 3, 10 June 2011, page S205, XP008174607
- MIKI NAKAMURA ET AL.: 'SELDI-TOF MS Proteome Kaiseki ni yoru Daichogan no Oxaliplatin Kanjusei Biomarker no Tansaku' JAPAN SOCIETY OF CLINICAL ONCOLOGY GAKUJUTSU SHUKAI vol. 52 ND, 15 July 2014, page LL-4, XP009500490

## Description

### Technical Field

The present invention relates to a marker for use in determination of the sensitivity of a cancer patient to an anti-cancer agent, which marker can determine whether or not the cancer of the patient has a therapeutic response to the anti-cancer agent, and to application of the marker.

### Background Art

Anti-cancer agents include various types of agents such as an alkylating agent, a platinum agent, an antimetabolite, an anti-cancer antibiotic, and an anti-cancer plant alkaloid. These anti-cancer agents may be effective for some types of cancers, or may not be effective for other types of cancers. However, it is known that even if an anti-cancer agent has been recognized to be effective for a certain type of cancer, the anti-cancer agent may be effective for the cancer, or may not be effective for the cancer, depending on individual patients. The factor of whether or not an anti-cancer agent is effective for the cancer of each of such individual patients, is referred to as sensitivity to the anti-cancer agent.

Oxaliplatin, (SP-4-2)-[(1R,2R)-cyclohexane-1,2-diamine-κN, κN'][ethanedio ato(2-)-κO¹, κO²]platinum (IUPAC), is a third-generation platinum-based complex antineoplastic drug. The action mechanism is believed to be based on inhibition of DNA synthesis, or protein synthesis, through the formation of crosslinks with DNA bases similarly to cisplatin (CDDP) or carboplatin (CBDCA), which are precedent drugs. However, oxaliplatin (L-OHP) exhibits an antitumor effect even against colorectal cancer, against which CDDP or CBDCA is ineffective, and oxaliplatin exhibits an antitumor spectrum different from that of conventional platinum-based complex antineoplastic drugs. In the United States, oxaliplatin for use in combination with fluorouracil (5-FU) /levofolinate (LV) was approved in January, 2004, as a first line treatment for metastatic colorectal cancer, and also in Japan, oxaliplatin for use in combination with continuous intravenous administration of levofolinate and fluorouracil (FOLFOX4 method) against "incurable and unresectable advanced/recurrent colorectal cancer" was listed in the National Health Insurance price list in April, 2005. Regarding the treatment of advanced/recurrent colorectal cancer, the survival rate provided by 5-FU/LV therapy that was conducted until the first half of the 1990's was 10 to 12 months, whereas the survival time provided by the FOLFOX therapy combined with oxaliplatin was 19.5 months, which is almost twice the survival time of the former. Furthermore, in August, 2009, "post-operative adjuvant chemotherapy for colon cancer" based on the same use of oxaliplatin in combination with continuous intravenous administration of levofolinate and fluorouracil was added to the efficacy and effectiveness. Thus, oxaliplatin is a drug that is promising for extended use and benefits in colorectal cancer patients.

However, even so, the response rate of the FOLFOX therapy against advanced/recurrent colorectal cancer is about 50%; in other words, it implies that the FOLFOX therapy is ineffective for half the number of those patients who have received treatment. Also, use of oxaliplatin leads to neutropenia as well as high-frequency peripheral neuropathy, and although these are not fatal side effects, these serve as a factor which makes it difficult to continue treatment. Therefore, when a biomarker, with which can predict which patients can expect to have an efficacy (responders) and which patients cannot (non-responders), before the initiation of treatment, to diagnose therapeutic response in an early stage, is used, a chemotherapy treatment with high effectiveness and high safety can be realized.

Furthermore, since a treatment schedule for cancer chemotherapy generally takes a long period of time, monitoring over time of sensitivity to an anti-cancer agent during continuation of treatment enables determination of whether treatment should be continued. Not only this leads to reduction of patient's burden or adverse side effects, but this is also considered useful even from the viewpoint of medical economics. In order to predict therapeutic response in individual patients, and to realize "personalized treatment", by which a diagnosis may be made early and appropriate medicament or treatment regimen may be selected, establishment of a biomarker which enables prediction of the efficacy of an anti-cancer agent such as oxaliplatin or an early diagnosis of therapeutic response, is urgent.

From such a point of view, the inventors of the present invention conducted a search for markers for determining sensitivity to an anti-cancer agent by culturing a plurality of human cancer cell lines , measuring drug sensitivities of these cancer cell lines, exposing these cell lines having different drug sensitivities to a drug, comprehensively analyzing the change in expression over time of intracellular proteins after exposure to the drug using a surface-enhanced laser desorption/ionization time-of-flight mass spectrometer (SELDI-TOF MS), making a comparison between the results and drug sensitivity, and analyzing the results. Thus, the inventors reported several markers (Patent Literatures 1 to 3). Martinez-Balibrea et al, 2009 (Int J Cane, 124(12), 2905 - 2910) discloses that the levels of ATP7B can be used to predict the therapeutic outcome of colorectal cancer to treatment with oxaliplatin/5FU.

Rasmussen et al, 2013 (Mol Oncol, 7(3), 637 - 646) discloses that the levels of some miRNA (miR-625-3p) are up-regulated in colorectal cancer patients not responding to oxaliplatin based treatment. EP2241334 discloses that the levels of protein A2 and protein A3 are associated with sensitivity of colon cancer cell lines to oxaliplatin. US2008096235 discloses several markers, such as e.g. fructose-bisphosphate aldolase A, the level of which is indicative of response of cancer cells to platinum-based anti-cancer agents.

US2007212738 discloses a method to predict whether a tumor will respond to treatment with an EGFR kinase inhibitor, said method comprising the measurement of epithelial biomarkers.

Vermont et al, 2011 (Cane Lett, 313(2), 167 - 180) discloses that lunasin potentiates the effect of oxaliplatin in colorectal cancer
(i.e. an anti-cancer sensitivity enhancer). D6 does not refer to TALDO.

Ma et al, 2009 (Electrophoresis, 30(15), 2591 - 2599), discloses measurement of TALDO via ELISA as a serum marker for colorectal cancer. WO2012172370 discloes measurement of TALDO.

### Citation List

### Patent Literature

Patent Literature 1: WO 2009/096196
Patent Literature 2: WO 2011/052748
Patent Literature 3: WO 2011/052749

### Summary of the Invention

### Problems to be Solved by the Invention

However, the markers that were previously reported have not yet been put to practical use as markers for determining sensitivity to an anti-cancer agent, and there is a demand for further development of new markers.

Therefore, it is an object of the present invention to provide a novel marker for determining sensitivity to an anti-cancer agent.

### Means for Solving the Problems

Thus, the inventors of the present invention first classified cancer cell lines into three classes, namely, a high-sensitivity type, a moderate-sensitivity type, and a low-sensitivity type, on the basis of the extent of sensitivity to an anti-cancer agent (oxaliplatin; L-OHP), and conducted an investigation on proteins, each of which showed a difference in the amount of expression of the protein that was expressed in high-sensitivity cell lines and low-sensitivity cell lines, by means of two-dimensional differential gel electrophoresis (2D-DIGE). As a result, the inventors found spots with different intracellular expression levels in the high-sensitivity cell lines and the low-sensitivity cell lines, analyzed the spots by LC-MS/MS, and performed database retrieval. Thereby, the inventors identified ten kinds of proteins. Next, the inventors examined the correlation between the amounts of expression of these proteins in three classes of cell lines such as a high-sensitivity cell line, a moderate-sensitivity cell line and a low-sensitivity cell line, and the IC₅₀ value of an anti-cancer agent. Thus, the inventors found that six proteins, namely, prohibitin (PHB), annexinA5 (ANXA5), annexinA1 (ANXA1), transaldolase (TALDO), complement component 1Q subcomponent-binding protein (C1QBP), and inorganic pyrophosphatase (IPYR), have high correlation.

Furthermore, an investigation was conducted on proteins, each of which showed a difference in the amount of expression of the protein that was expressed in high-sensitivity cell lines and low-sensitivity cell lines, by means of surface-enhanced laser desorption/ionization time-of-flight mass spectrometer (SELDI-TOF MS). Meanwhile, in order to conduct an extensive search for proteins showing differences in the amounts of expression, an investigation was made by means of SELDI-TOF MS using both a cationic chip and an anionic chip, and using, as matrices, SPA (EAM: a saturated solution of sinapinic acid in a 50% ACN/0. 5% TFA solution) for high molecular weight substances and CHCA (α-cyano-4-hydroxycinnamic acid) for low molecular weight substances. In an intracellular protein extraction process, intracellular proteins were extracted using a cell lysate directly without scraping with a rubber policeman, in order to avoid stimulation of cells and activation of intracellular proteins. As a result, the inventors obtained information on the estimated molecular weights and isoelectric points for those proteins showing differences in the amount of intracellular expression in high-sensitivity cell lines and low-sensitivity cell lines. These proteins were subjected to database retrieval, and thus cytochrome c oxidase subunit 5A (COX5A) was identified. Furthermore, two-dimensional gel electrophoresis of cell extracts of high-sensitivity cell lines and low-sensitivity cell lines was performed, and spots that were in the estimated molecular weight and isoelectric point ranges and showed difference in the amount of expression, were found. Those spots were analyzed by LC-MS/MS and were subjected to database retrieval. Thereby, retinol-binding protein 1 (CRBP1) was identified.

The inventors conducted a further investigation based on such findings, and as a result, the inventors found that when the concentrations of any of the above-mentioned proteins in a biological sample derived from a cancer patient are measured, whether the cancer of the cancer patient has sensitivity to an anti-cancer agent can be determined; when the variations in expression of any of these substances are employed as an index, screening of an anti-cancer agent sensitivity enhancer is enabled; and when the above-mentioned anti-cancer agent sensitivity enhancer is used in combination with an anti-cancer agent that is a target for sensitivity enhancement, the therapeutic effect of the anti-cancer agent is remarkably enhanced. Thus, the inventors completed the present invention.

That is, the present invention provides the following items [1] to [6].
[1] Use of TALDO as a marker for determining sensitivity to an anti-cancer agent, which anti-cancer agent is oxaliplatin or a salt thereof and which cancer is colorectal cancer.
[2] A method for determining sensitivity to an anti-cancer agent, which anti-cancer agent is oxaliplatin or a salt thereof and which cancer is colorectal cancer, the method comprising a step of measuring an amount of TALDO in a biological sample derived from a cancer patient.
[3] The determination method according to [2], further comprising a step of determining the sensitivity of the cancer patient to oxaliplatin or a salt thereof by comparing the measurement result with a control level.
[4] The determination method according to [2] or [3], wherein the biological sample is a biological sample derived from a cancer patient to which oxaliplatin or a salt thereof has been administered.
[5] Use of a kit for performing the determination method according to any one of [2] to [4], the kit comprising a protocol for measuring the amount of TALDO in a biological sample derived from a cancer patient.
[6] A screening method for an anti-cancer agent sensitivity enhancer, which anti-cancer agent is oxaliplatin or a salt thereof and which cancer is colorectal cancer, the method comprising employing, as an index, variation in expression of TALDO in a cancer cell line or a biological sample derived from a tumor-bearing animal in the presence of oxaliplatin or a salt thereof.

### Effects of the Invention

When the marker for determining sensitivity to an anti-cancer agent of the present invention is used, the sensitivity of individual patients to an anti-cancer agent can be reliably determined before the initiation of treatment or in an early stage after the initiation of treatment, and as a result, selection of an anti-cancer agent which can provide a high therapeutic effect is enabled. Furthermore, since use of an anti-cancer agent which does not provide an effect can be avoided, unnecessary adverse side effects can be avoided. Furthermore, since a therapeutic schedule using an anti-cancer agent requires a long period of time, when the sensitivity to the anti-cancer agent is determined for each therapeutic cycle during continuation of treatment, an evaluation over time of the sensitivity of the relevant cancer to the anti-cancer agent is enabled, and determination of whether treatment should be continued or not can be made. As a result, progress of cancer associated with continued administration of an anti-cancer agent which does not provide a therapeutic effect, and increase in adverse side effects can be prevented, and this also leads to reduction of the burden of patients and reduction of medical expenses.

Furthermore, when this marker is used, drugs that enhance anti-cancer agent sensitivity can be selected through screening. Thus, when a target anti-cancer agent is used in combination with an anti-cancer agent sensitivity enhancer, the cancer treatment effect is dramatically enhanced. A reagent for measuring the marker for determining sensitivity to an anti-cancer agent is useful as a reagent for determining sensitivity to an anti-cancer agent.

### Brief Description of the Drawings

Fig. 1 shows results for 2D-DIGE. Encircled parts in the diagram show sixteen spots that have been selected.
Fig. 2 illustrates a relation between an amount of expression of PHB and oxaliplatin sensitivity (IC₅₀ value) in cancer cells. The IC₅₀ values plot, from the left-hand side, the results for SW480, Ls174T, Lovo, SW620, HCT116, HCT15, HT29, DLD-1, and WiDr.
Fig. 3 illustrates a relation between an amount of expression of C1QBP and the oxaliplatin sensitivity (IC₅₀ value) in cancer cells. The IC₅₀ values plot, from the left-hand side, the results for SW480, Ls174T, Lovo, SW620, HCT116, HCT15, HT29, DLD-1, and WiDr.
Fig. 4 illustrates a relation between an amount of expression of ANXA5 and the oxaliplatin sensitivity (IC₅₀ value) in cancer cells. The IC₅₀ values plot, from the left-hand side, the results for SW480, Ls174T, Lovo, SW620, HCT116, HCT15, HT29, DLD-1, and WiDr.
Fig. 5 illustrates a relation between an amount of expression of ANXA1 and the oxaliplatin sensitivity (IC₅₀ value) in cancer cells. The IC₅₀ values plot, from the left-hand side, the results for SW480, Ls174T, Lovo, SW620, HCT15, HT29, DLD-1, and WiDr.
Fig. 6 illustrates a relation between an amount of expression of TALDO and the oxaliplatin sensitivity (IC₅₀ value) in cancer cells. The IC₅₀ values plot, from the left-hand side, the results for SW480, Ls174T, Lovo, SW620, HCT116, HCT15, HT29, DLD-1, and WiDr.
Fig. 7 illustrates a relation between an amount of expression of IPYR and the oxaliplatin sensitivity (IC₅₀ value) in cancer cells. The IC₅₀ values plot, from the left-hand side, the results for SW480, Ls174T, Lovo, SW620, HCT116, HCT15, HT29, DLD-1, and WiDr.

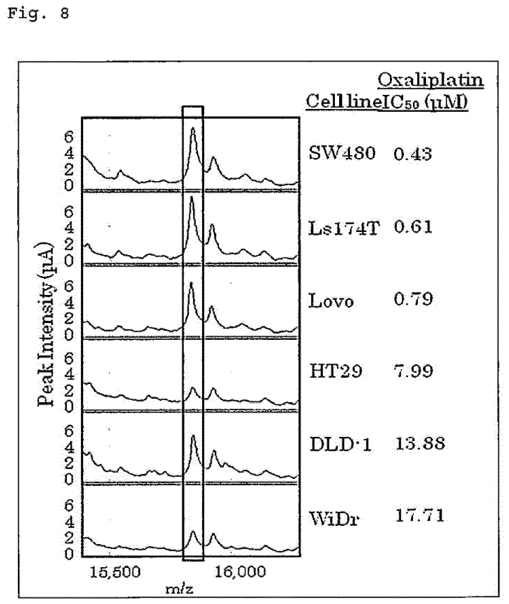

Fig. 8 illustrates SELDI-TOF MS results for candidate protein A.
Fig. 9 illustrates prediction for an isoelectric point of candidate protein A.
Fig. 10 illustrates SELDI-TOF MS results for candidate protein B.
Fig. 11 illustrates prediction for an isoelectric point of candidate protein B.
Fig. 12 illustrates results of two-dimensional gel electrophoresis for an L-OHP high-sensitivity cell line and a low-sensitivity cell line that was conducted in order to identify molecular weight and the isoelectric point estimated by SELDI-TOF MS.
Fig. 13 shows a comparison of various peak intensity analysis results obtained by Western blotting and SELDI-TOF MS of m/z 15,850 (CRBP1).
Fig. 14 shows a comparison of various peak intensity analysis results obtained by Western blotting and SELDI-TOF MS of m/z 12,506 (COX5A).

### Modes for Carrying out the Invention

The marker for determining sensitivity to an anti-cancer agent according to the present invention is TALDO. In regard to eight kinds of proteins, namely, PHB, ANXA5, ANXA1, TALDO, C1QBP, IPYR, CRBP1 and COX5A, as disclosed in the following Examples, an investigation was conducted on the differences in the amounts of expression of the proteins expressed in cell lines having high sensitivity to an anti-cancer agent and cell lines having low sensitivity thereto, using 2D-DIGE or SELDI-TOF MS, and as a result, it was found that the amounts of expression of four proteins, namely, PHB, C1QBP, CRBP1 and COX5A, increased in the high-sensitivity cell lines. It was also found that the amounts of expression of four proteins, namely, ANXA5, ANXA1, TALDO and IPYR, increased in the low-sensitivity cell lines. Therefore, these eight proteins are useful as markers for determining sensitivity to an anti-cancer agent, particularly as markers for determining sensitivity to oxaliplatin.

It is known that PHB is a tumor suppressor gene (JP-A-5-271294) and can be used as a prostate cancer marker (JP-A-2012-196211); however, it is not known at all that PHB can be used as a marker for determining sensitivity to an anti-cancer agent, and that the concentration of PHB increases in cancer cells having high anti-cancer agent sensitivity.

ANXA5 and ANXA1 can be used for the diagnosis of cancer in the genitourinary tract and the intestinal tract (JP-A-2008-545634) ; however, it is not known at all that ANXA5 and ANXA1 can be used as markers for determining sensitivity to an anti-cancer agent, and that the concentrations of ANXA5 and ANXA1 increase in cancer cells having low anti-cancer agent sensitivity.

There is no report on the use of TALDO as a cancer marker, and it is not known at all that TALDO can be used as a marker for determining sensitivity to an anti-cancer agent, and that the concentration of TALDO increases in cancer cells having low anti-cancer agent sensitivity.

It is known that C1QBP can be used as a diagnosis marker for renal cell cancer (JP-A-2006-514554); however, it is not known at all that C1QBP can be used as a marker for determining sensitivity to an anti-cancer agent, and that the concentration of C1QBP increases in cancer cells having high anti-cancer agent sensitivity.

It is known that IPYR can be used for a method of diagnosing colorectal cancer in vitro (JP-A-2008-502889); however, it is not known at all that IPYR can be used as a marker for determining sensitivity to an anti-cancer agent, and that the concentration of IPYR increases in cancer cells having low anti-cancer agent sensitivity.

It is known that CRBP1 promotes a cell proliferation suppressing action caused by retinol in breast cancer or colorectal cancer (Kaleagasioglu F, et al., Arzneimittelforschung (1993), 43(4): 487-90); however, it is not known at all that CRBP1 can be used as a marker for determining sensitivity to an anti-cancer agent, and that the concentration of CRBP1 increases in cancer cells having high anti-cancer agent sensitivity.

It is known that in a mouse colorectal cancer model, the expression level of COX5A in cancer cells is lower compared to the expression level in normal cells (Yasui Y, et al., J. Carcinog. , (2009) 8:10). Furthermore, in paragraph (0068) of Patent Literature 3, COX5A is mentioned as one of candidates for Protein G. However, in Patent Literature 3, Protein G shows an increased concentration in cell lines with low anti-cancer agent sensitivity compared to cell lines with high anti-cancer agent sensitivity, and exhibits results that are opposite to those of the present invention. Therefore, it may be considered that Protein G of Patent Literature 3 is not COX5A. For this reason, it can be said that it is not known at all that COX5A can be used as a marker for determining sensitivity to an anti-cancer agent, and that the concentration of COX5A increases in cancer cells having high anti-cancer agent sensitivity.

The anti-cancer agent that is a target of the marker for determining sensitivity to an anti-cancer agent of the present invention is oxaliplatin or a salt thereof.

In order to determine anti-cancer agent sensitivity using the marker for determining sensitivity to an anti-cancer agent of the present invention, determination can be made by measuring the amount of TALDO in a biological sample (specimen) derived from a cancer patient, and more particularly, comparing the measurement results with the control levels (for example, standard concentrations, and concentrations of the markers for determining sensitivity to an anti-cancer agent of the present invention prior to anti-cancer agent administration).

Here, cancer patients include test subjects who have cancer, and test subjects who previously had cancer. Examples of the biological sample include blood, serum, plasma, a cancer tissue biopsy specimen, an operatively extracted cancer specimen, faeces, urine, ascitic fluid, pleural fluid, cerebrospinal fluid, and sputum, and plasma is particularly preferred.

The target cancer of the present invention is colorectal cancer.

Regarding the measurement means for the molecules selected from the group consisting of PHB, ANXA5, ANXA1, TALDO, C1QBP, IPYR, CRBP1 and COX5A in a specimen, the molecules can be measured by, for example, gel electrophoresis (for example, 2D-DIGE), mass spectrometry (for example, SELDI-TOFMS, LC/MS, or LC/MS/MS), or an immunoassay (for example, immunoblotting or ELISA).

Here, measurement by 2D-DIGE and SELDI-TOF MS can be carried out by the methods described in the following Examples. Furthermore, regarding measurement made by mass spectrometry such as LC/MS or LC/MS/MS, molecules selected from the group consisting of PHB, ANXA5, ANXA1, TALDO, C1QBP, IPYR, CRBP1 and COX5A can be measured by performing a quantitative analysis according to a conventional method. Furthermore, in regard to an immunoassay method, a measurement method of using antibodies to the molecules selected from the group consisting of PHB, ANXA5, ANXA1, TALDO, C1QBP, IPYR, CRBP1 and COX5A is preferred. The antibodies that can be used with respect to the molecules selected from the group consisting of PHB, ANXA5, ANXA1, TALDO, C1QBP, IPYR, CRBP1 and COX5A may be monoclonal antibodies, or may be polyclonal antibodies. More specific examples of the immunoassay include radioimmunoassay, enzyme immunoassay, fluorescence immunoassay, luminescence immunoassay, immunoprecipitation, immunonephelometry, Western blotting, immunostaining, and immunodiffusion. Preferred examples include Western blotting and enzyme immunoassay, and particularly preferred examples include Western blotting and enzyme-linked immunosorbent assay (ELISA) (for example, sandwich ELISA).

With regard to ANXA5, ANXA1, TALDO and IPYR, in the case where the target anti-cancer agent is oxaliplatin or a salt thereof, in order to determine the sensitivity to the anti-cancer agent, the amounts of ANXA5, ANXA1, TALDO and/or IPYR, for example, the concentrations thereof, in a biological sample derived from a cancer patient may be measured before administration of the anti-cancer agent or in an early stage after administration. When the concentration (s) is lower than a predetermined standard concentration(s), the cancer can be determined to have sensitivity to the target anti-cancer agent, and thus these markers for determining sensitivity to an anti-cancer agent can be used as markers for active continuation of treatment in a patient who can be expected to receive therapeutic effects . On the other hand, when the concentration(s) is higher than a predetermined standard concentration(s), the cancer is determined to have no sensitivity to the target anti-cancer agent. When the cancer has no sensitivity to the target anti-cancer agent, efficacy of the anti-cancer agent cannot be expected. If administration of such an ineffective anti-cancer agent is performed or continued, there is a risk for progress of cancer and an increase in adverse side effects. As such, the marker for determining sensitivity to an anti-cancer agent according to the present invention can be used as a marker for actively continuing treatment in patients who can be expected to receive therapeutic effects, and can also be used as a marker for avoiding the progress of cancer and an increase in adverse side effects, which are associated with continued administration of an ineffective anti-cancer agent.

With regard to PHB, C1QBP, CRBP1 and COX5A, in the case where the target anti-cancer agent is oxaliplatin or a salt thereof, in order to determine the sensitivity to the anti-cancer agent, the amounts of PHB, C1QBP, CRBP1 and/orCOX5A, for example, the concentrations thereof, in a biological sample derived from a cancer patient may be measured before administration of the anti-cancer agent or in an early stage after administration. When the concentration (s) is higher than a predetermined standard concentration(s), the cancer can be determined to have sensitivity to the target anti-cancer agent, and thus these markers for determining sensitivity to an anti-cancer agent can be used as markers for active continuation of treatment in a patient who can be expected to receive therapeutic effects. On the other hand, when the concentration(s) is lower than a predetermined standard concentration(s), the cancer can be determined to have no sensitivity to the target anti-cancer agent. When the cancer has no sensitivity to the target anti-cancer agent, efficacy of the anti-cancer agent cannot be expected. If administration of such an ineffective anti-cancer agent is performed or continued, there is a risk for progress of cancer and an increase in adverse side effects. As such, the marker for determining sensitivity to an anti-cancer agent according to the present invention can be used as a marker for actively continuing treatment in patients who can be expected to receive therapeutic effects, and can also be used as a marker for avoiding the progress of cancer and an increase in adverse side effects, which are associated with continued administration of an ineffective anti-cancer agent.

In order to perform the method for determining sensitivity to an anti-cancer agent of the present invention, it is preferable to use a kit comprising a protocol for measuring TALDO in a specimen. The kit comprises a reagent for measuring TALDO, and a protocol (for example, a method of using the measurement reagent, and references for determining the presence or absence of anti-cancer agent sensitivity). The references include, for example, the standard concentrations of TALDO, concentrations that are considered to be high, concentrations that are considered to be low, factors that affect the measurement results, and the extent of the influence. These concentrations can be set as appropriate for each target anti-cancer agent. Determination can be made as described above, using these references.

Furthermore, when the variation in expression of TALDO in a biological sample derived from a cancer cell line or a tumor-bearing animal in the presence of an anti-cancer agent, is employed as an index, screening of an anti-cancer agent sensitivity enhancer is enabled.

That is, the anti-cancer agent sensitivity enhancer can be selected through screening by performing a step of adding or administering an anti-cancer agent and a test substance to a cancer cell line or a tumor-bearing animal, and measuring the amount of expression of TALDO in a biological sample derived from the cancer cell line or the tumor-bearing animal at a gene level or at a protein level; and a step of selecting a test substances that enhances sensitivity of the cancer cell line or tumor-bearing animal to the anti-cancer agent based on the variation in the amount of expression.

For example, with regard to TALDO, and the target anti-cancer agent is oxaliplatin or a salt thereof, when the variation in expression, more specifically suppression of expression, of any of these proteins is employed as an index, an anti-cancer agent sensitivity enhancer can be selected through screening. That is, a substance which suppresses expression of any of these proteins in vitro or in vivo enhances anti-cancer agent sensitivity. For example, under in vitro conditions, a substance which decreases the concentrations of any of these proteins in the presence of an anti-cancer agent in various cancer cell lines, is a substance that enhances the sensitivity of the cancer cell lines to the anti-cancer agent (anti-cancer agent sensitivity enhancer). Furthermore, under in vivo conditions, a substance which decreases the concentrations of any of these proteins before and after the administration of an anti-cancer agent in a tumor-bearing animal, is a substance that enhances the sensitivity of the tumor-bearing animal to the anti-cancer agent (anti-cancer agent sensitivity enhancer).

When the thus-obtained anti-cancer agent sensitivity enhancer is used in combination with an anti-cancer agent which is a sensitivity enhancement target of the enhancer, the therapeutic effect of the anti-cancer agent is dramatically enhanced. The form of the combination of the anti-cancer agent sensitivity enhancer and the anti-cancer agent which is a sensitivity enhancement target of the enhancer, may be a single composition including both of those components, or may be a combination of separate preparations of the respective components. Those components may also be administered respectively through different routes of administration. Examples of the target anti-cancer agent to be used herein include, as described above, oxaliplatin, cyclophosphamide, ifosfamide, thiotepa, melphalan, busulfan, nimustine, ranimustine, dacarbazine, procarbazine, temozolomide, cisplatin, carboplatin, nedaplatin, methotrexate, pemetrexed, fluorouracil, tegafur/uracil, doxifluridine, tegafur/gimeracil/oteracil, capecitabine, cytarabine, enocitabine, gemcitabine, 6-mercaptopurine, fludarabine, pentostatin, cladribine, hydroxyurea, doxorubicin, epirubicin, daunorubicin, idarubicin, pirarubicin, mitoxantrone, amrubicin, actinomycinD, bleomycin, pepleomycin, mytomycinC, aclarubicin, zinostatin, vincristine, vindesine, vinblastine, vinorelbine, paclitaxel, docetaxel, irinotecan, irinotecan active metabolite (SN-38), nogitecan (topotecan), etoposide, prednisolone, dexamethasone, tamoxifen, toremifene, medroxyprogesterone, anastrozole, exemestane, letrozole, rituximab, imatinib, gefitinib, gemtuzumab/ozogamicin, bortezomib, erlotinib, cetuximab, bevacizumab, sunitinib, sorafenib, dasatinib, panitumumab, asparaginase, tretinoin, arsenictrioxide, salts thereof, and active metabolites thereof. Among these, platinum-based complex anti-cancer agents are preferred, and oxaliplatin or a salt thereof is particularly preferred.

### Examples

Next, the present invention will be described in more details by way of Examples.

### Test Example 1

Measurement of sensitivity to oxaliplatin of human colorectal cancer cell lines

### (1) Method

### (a) Cells used

Nine human colorectal cancer cell lines (SW480, Ls174T, Lovo, SW620, HCT116, HCT15, HT29, DLD-1, and WiDr) were obtained from the following sources (Table 1).

Culture was carried out under the conditions of φ100 mm/Tissue Culture Dish (IWAKI), culture medium (D-MEM, 2 mM Glutamine, 10% Fetal Bovine Serum), 37°C, and 5% CO₂.

**[Table 1]**

| Nine kinds of human colorectal cancer cell lines | | | | |
|---|---|---|---|---|
| Cell line name | Bank from which cell line was obtained | Deposition(or supplier) | Resource No. or the like | Lot No. or the like |
| SW480 | ECACC | Sumitomo Dainippon Pharma Co., Ltd. | EC-87092801 | 02/A/063 |
| Ls174T | TKG | Cell Resource Center for Biomedical Research, Institute of Development, Aging and Cancer, Tohoku University | TKG0406 | I-4468 |
| Lovo | ECACC | Sumitomo Dainippon Pharma Co., Ltd. | EC-8706101 | - |
| SW620 | ATCC | Summit Pharmaceuticals International Corporation | CCL-227 | 2324584 |
| HCT116 | ATCC | Yakult Honsha Co., Ltd. | CCL-247 | - |
| HCT15 | TKG | Cell Resource Center for Biomedical Research, Institute of Development, Aging and Cancer, Tohoku University | TKG0504 | 1-4608 |
| HT29 | ATCC | Yakult Honsha Co., Ltd. | HTB-38 | - |
| DLD-1 | ECACC | Sumitomo Dainippon Pharma Co., Ltd. | EC-90102540 | 00/J/025 |
| WiDr | ECACC | Sumitomo Dainippon Pharma Co., Ltd. | EC-85111501 | 00/H/001 |

### (b) Drug

Oxaliplatin (L-OHP) bulk powders were obtained from Wako Pure Chemical Industries, Ltd. and Yakult Honsha Co., Ltd.

### (c) Method for measuring sensitivity to oxaliplatin of human colorectal cancer cell lines

The aforementioned nine human colorectal cancer cell lines were respectively inoculated onto a 96well plate at a density of 1,500 cells/well, and after 24 hours, oxaliplatin was added thereto. The cell survival rate after 48 hours of drug exposure was evaluated by the MTS assay (CellTiter96™ AQueous One Solution Cell Proliferation Assay, Promega), and the IC₅₀ values were determined from the absorbance values. Regarding the drug exposure conditions, eleven conditions such as Control (0 µM), 0.001 µM, 0.01 µM, 0.1 µM, 0.3 µM, 1 µM, 3 µM, 10 µM, 30 µM, 100 µM, and 1,000 µM were used. The evaluation of sensitivity was performed three times with cells of different passage numbers, by measuring three samples each for the respective cell lines, drug exposure time, and drug exposure conditions in a single time test. An analysis was carried out based on the survival rate calculated from the results of the MTS assay.

### (2) Results

The IC₅₀ values had the values indicated in Table 2. From the results of Table 2, SW480, Ls174T and Lovo were classified as oxaliplatin-high-sensitivity cell lines; SW620, HCT116 and HCT15 were classified as oxaliplatin-moderate-sensitivity cell lines; and HT29, DLD-1 and WiDr were classified as oxaliplatin-low-sensitivity cell lines.

**[Table 2]**

| Colorectal cancer cell line | IC₅₀ value (µM) |
|---|---|
| SW480 | 0.43±0.13 |
| Ls174T | 0.61±0.11 |
| Lovo | 0.79±0.18 |
| SW620 | 1.14±0.74 |
| HCT116 | 1.15±0.34 |
| HCT15 | 1.45±0.44 |
| HT29 | 7.99±2.66 |
| DLD-1 | 13.88±6.29 |
| WiDr | 17.71±8.39 |

### Example 1

Search for biomarkers for predicting sensitivity to oxaliplatin by 2D-DIGE

### (1) Cells used

SW480, Ls174T and Lovo, which were classified as oxaliplatin-high-sensitivity cell lines in Test Example 1, and HT29, DLD-1 and WiDr, which were classified as oxaliplatin-low-sensitivity cell lines in Test Example 1, were used.

### (2) Method for extracting intracellular proteins

The culture medium was removed from the dish, and the cells were washed three times with ice-cold PBS. Subsequently, in order to avoid stimulation of cells and activation of intracellular proteins, a cell lysis solution (2 M thiourea, 7 M Urea, 4% CHAPS, 50 mM Tris-HCl, pH 9) was directly added to the cells to thereby lyse the cells, and the lysate was transferred into a 1.5 mL microtube. The cell lysate was subjected to ultrasonication under ice cooling, and then the resultant was centrifuged for 10 minutes at 4°C at 13, 000×rpm. Thus, a supernatant was collected. A quantitative analysis of proteins was performed using a 2D Quant kit (GE Healthcare), and the supernatant was adjusted to 5 mg/mL using the cell lysis solution. Subsequently, the resultant was dispensed and stored at -80°C until use for an analysis.

### (3) Labeling of proteins by CyDye™ DIGE Flour minimal dyes

Intracellular proteins were labeled using CyDye DIGE fluors, minimal labeling kit (GE Healthcare). 50 µg of each of the cell lysates was labeled with 400 pmol of reagents (Cy2, Cy3, or Cy5), and each cell lysate was sufficiently mixed. The labeled cell lysate was lightly centrifuged with a centrifuge, and was left to stand for 30 minutes in a dark place at 4°C. 10 mM lysine was added thereto to stop the reaction. The solution was mixed and lightly centrifuged, and the resultant was left to stand for 10 minutes in a dark place at 4°C. The resultant was stored at -80°C until use.

### (4) Isoelectric focusing (first dimension)

Samples applied to each gel are presented in Table 3.

For the internal standard, a mixture of equal amounts of all of the cell lysates was used.

For each gel, 50 µg (12 µL) of each of the protein samples labeled in section (3) was transferred to a 1.5 mL microtube and mixed. 36 µL of 2× sample buffer (2 M thiourea, 7 M urea, 4% CHAPS, 2% PharmalytepH 3-10, 2% Destreak Reagent) was added to the mixed protein sample, and the mixture was left to stand on ice for 10 minutes . 150 µg of each sample solution was adjusted to 450 µL with a swelling solution (2 M thiourea, 7 M urea, 4% CHAPS, 2% PharmalytepH 3-10, 1% Destreak Reagent), and was centrifuged for 10 minutes at 4°C at 13, 000×rpm. 450 µL of the sample supernatant was transferred to each strip holder, Immobiline DryStrip pH3-10 NL, 24 cm (GE Healthcare) was layered onto the strip holder, and then isoelectric focusing was performed using Ettan IPGphor2. The conditions for the isoelectric focusing are presented in Table 4.

**[Table 3]**

| gel.no | Cy2 | Cy3 | Cy5 |
|---|---|---|---|
| 1 | Internal standard | SW480 | HT29 |
| 2 | Internal standard | Ls174T | DLD-1 |
| 3 | Internal standard | Lovo | WiDr |
| 4 | Internal standard | DLD-1 | SW480 |
| 5 | Internal standard | WiDr | LS174T |
| 6 | Internal standard | HT29 | Lovo |

**[Table 4]**

| Rehydrate | | | | | |
|---|---|---|---|---|---|
| 10Hr | | | | | |
| 20°C | | | | | |
| step | Voltage change pattern | | Voltage (V) | Time (hr) | kVhr |
| 1 | Step and Hold | | 500 | 1:00 | 0.5 |
| 2 | Gradient | | 1000 | 1:00 (8:00) | 0.8 (0.6) |
| 3 | Gradient | | 8000 | 3:00 | 13.5 |
| 4 | Step and Hold | | 8000 | 2:30-3:45 | 20-30 |
| 5 | Step and Hold | | 500 | 2:00 | |

### (5) SDS-PAGE (second dimension)

15% Acrylamide gel having a size of 24 cm was produced for SDS-PAGE. The Immobiline DryStrip that had been subjected to first dimension electrophoresis was placed on a 10 cm dish, the dish was filled with equilibration buffer A (50 mM Tris-HCl, pH 8.8, 6 M Urea, 30% glycerol, 1% SDS, 0.25% DTT, and BPB), and the Immobiline DryStrip was equilibrated by shaking the dish for 15 minutes. The buffer was replaced with equilibration buffer B (50 mM Tris-HCl pH 8.8, 6 M Urea, 30% glycerol, 1% SDS, 4.5% iodoacetamide, and BPB), and the Immobiline DryStrip was equilibrated by shaking the dish for 15 minutes. The equilibrated Immobiline DryStrip was placed on the SDS-PAGE gel, and electrophoresis was performed at 7 mA using an electrophoresis chamber (Tris/Tricin/SDS buffer).

Images were captured with Typhoon Trio (GE Healthcare), and the data were analyzed using DeCyder 2D Software Ver 6.5 (GE Healthcare). Thus, spots with P < 0.001 and having a difference in the amounts of expression of 1.3 times or more were analyzed.

### (6) Collection of spots

150 µg of a non-labeled internal standard was subjected to two-dimensional electrophoresis in the same manner as in sections (4) and (5).

Next, in order to collect the spots obtained in section (5), the gel was stained with silver using PlusOne™ Silver Staining Kit, Protein (GE Healthcare).

First, 250 mL of a fixing solution (30% ethanol, 10% glacial acetic acid) was added to the gel, and the gel was left to stand for 60 minutes. This operation was repeated two times, and thereby the gel was fixed. 250 mL of a sensitizing solution (30% ethanol, 4% sodium thiosulfate (5% w/v), 6.8% sodium acetate) was added to the fixed gel, and the gel was left to stand for 120 minutes to sensitize the gel. The gel was washed for 8 minutes using Milli-Q™ water, and this washing was repeated five times. 250 mL of a silver solution (10% silver nitrate solution (2.5% w/v)) was added to the washed gel, and the gel was reacted with silver for 60 minutes. The gel was washed for 1 minute using Milli-Q water, and this washing was repeated four times . 250 mL of a developing solution (2.5% sodium carbonate, 0.08% formaldehyde (37% w/v)) was added to the washed gel, and thereby the gel was developed for 2 to 5 minutes. 250 mL of a stop solution (1.46% EDTA-Na₂·2H₂O) was added thereto, the gel was left to stand for 45 minutes, and thus the reaction was stopped. The gel was washed for 30 minutes using Milli-Q water, and this washing was repeated two times. Subsequently, spots intended for analysis were collected using a spot picker (Gene World), and four spots per microtube were transferred into a 1.5 mL microtube.

### (7) Analysis of spots

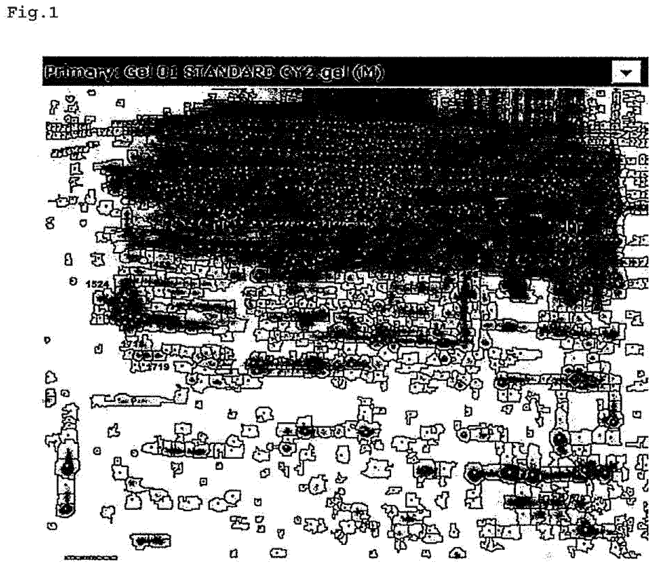

For the sixteen spots (encircledparts in Fig. 1) collected by the operations up to section (6), the spots were subjected to in-gel trypsin digestion of proteins according to a known method, and then the spots were analyzed (MS/MS analysis) using LCMS-IT-TOF (liquid chromatograph mass spectrometer, Shimadzu). The results thus obtained were subjected to MASCOT database retrieval.

As a result of database retrieval, six kinds of proteins, namely, prohibitin (PHB), annexinA5 (ANXA5), annexin A1 (ANXA1), transaldolase (TALDO), complement component 1Q subcomponent-binding protein (C1QBP), and inorganic pyrophosphatase (IPYR), were identified.

### Example 2

### Correlation between amounts of expression of six proteins in colorectal cancer cell lines and IC₅₀ values

For the six proteins identified in Example 1, the correlation between the amounts of expression in the nine kinds of colorectal cancer cell lines used in Test Example 1 and the IC₅₀ values calculated in Test Example 1 was examined.

### (1) Method

### (a) Cells used

The nine kinds of colorectal cancer cell lines described in Test Example 1 were used.

### (b) Extraction of intracellular proteins

The culture medium was removed from the dish, and the cells were washed three times with ice-cold PBS. Subsequently, in order to avoid stimulation of cells and activation of intracellular proteins, a cell lysis solution (9 MUrea, 2% CHAPS, 50 mM Tris-HCl, pH 9) was directly added to the cells to thereby lyse the cells, and the lysate was transferred into a 1.5 mL microtube. The cell lysate was subjected to ultrasonication under ice cooling, and then the resultant was centrifuged for 10 minutes at 4°C at 13,000×rpm. Thus, a supernatant was collected. A quantitative analysis of proteins was performed using a BCA Protein Assay Kit (Thermo), and the supernatant was adjusted to 5mg/mL using the cell lysis solution. Subsequently, the resultant was dispensed and stored at -80°C until use for an analysis.

### (c) Western blotting

The nine colorectal cancer cell lines described in Test Example 1 were respectively added in an amount of 10 µL/Lane (50 µg/Lane) to 10% acrylamide gel, and SDS-PAGE was performed using an electrophoresis chamber (Tris/Glycine/SDS buffer) at 20 mA per sheet. Transfer to a PVDF membrane (GE-Healthcare) was performed at 72 mA per sheet for 30 minutes using a semi-dry type blotter (BIO-RAD), and blocking was carried out for one hour at room temperature using 5% skimmed milk/TBS-T (Tris buffered saline with Tween20). The primary antibodies shown in Table 5 were added thereto, and a reaction was carried out overnight at 4°C.

**[Table 5]**

| Primary antibody name | Supplier | Amount of addition |
|---|---|---|
| Prohibitin-mitochondrial marker rabbit polyclonal | abcam, ab28172 | ×1/10000 |
| Annexin A5 rabbit polyclonal | abcam, ab14196 | ×1/5000 |
| Annexin A1 [5E4/1] mouse monoclonal | abcam, ab2487 | ×1/200 |
| Transaldolase 1 mouse polyclonal | abcam, ab67467 | ×1/200 |
| Complement component 1Q -binding protein goat polyclonal | santa cruz, sc-10258 | ×1/10000 |
| Pyrophosphatase 1 rabbit polyclonal | abcam, ab96099 | ×1/10000 |
| GAPDH mouse monoclonal (6C5) | santa cruz, sc-32233 | ×1/10000 |

The membrane was washed three times with TBS-T, subsequently the secondary antibodies shown in Table 6 were added thereto, followed by shaking for 1 hour at room temperature. The membrane was washed with TBS-T, and then the membrane was reacted with ELC Prime Western Blotting Detection (GEHealthcare) . Images were captured using LAS4000 mini (GE Healthcare), and the images were analyzed.

**[Table 6]**

| Secondary antibody name | Supplier | Amount of addition |
|---|---|---|
| anti-mouse IgG | GE Healthcare | ×1/20000 |
| anti-rabbit IgG | GE Healthcare | ×1/20000 |
| anti-goat IgG | Abcam | ×1/20000 |

### (2) Results

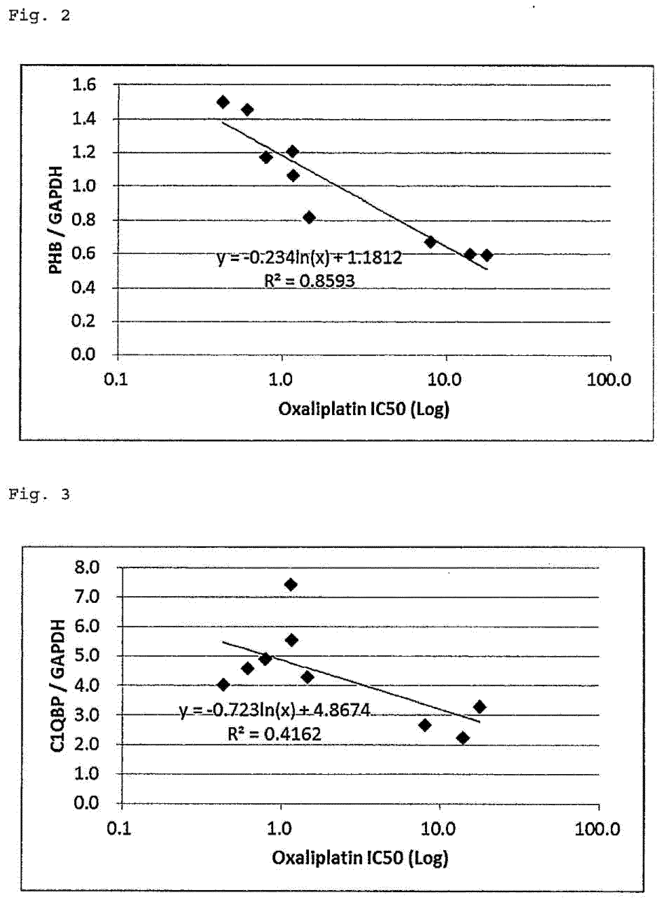

PHB and C1QBP had large amounts of expression in the oxaliplatin-high-sensitivity cell lines, and had small amounts of expression in the low-sensitivity cell lines. Thus, these proteins exhibited high positive correlations (Fig. 2 and Fig. 3) .

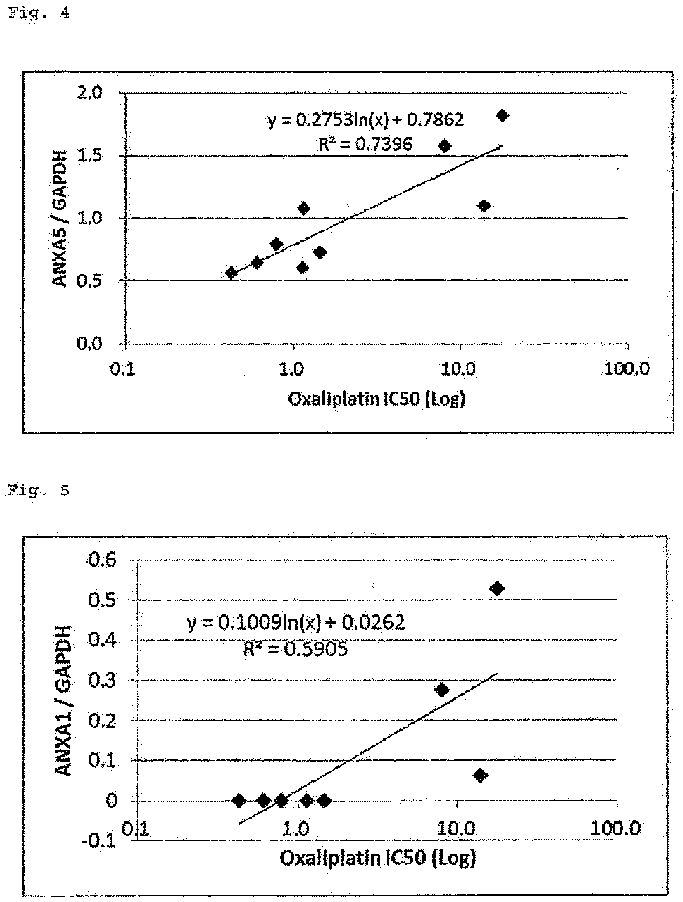

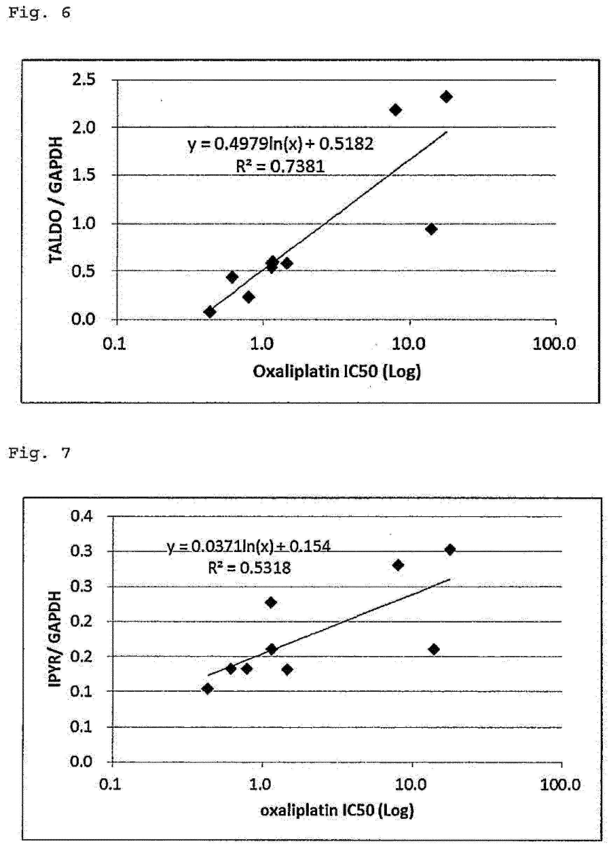

ANXA5, ANXA1, TALDO and IPYR had small amounts of expression in the oxaliplatin-high-sensitivity cell lines, and had large amounts of expression in the low-sensitivity cell lines . Thus, these proteins exhibited high negative correlations (Fig. 4 to Fig. 7).

### Example 3

### Search for biomarkers for predicting sensitivity to oxaliplatin by using SELDI-TOF MS

### (1) Method

### (a) Cells used

SW480, Ls174T and Lovo, which were classified as oxaliplatin-high-sensitivity cell lines in Test Example 1, and HT29, DLD-1 and WiDr, which were classified as oxaliplatin-low-sensitivity cell lines in Test Example 1, were used.

### (b) Extraction of intracellular proteins

Extraction was performed by a method similar to that used in Example 2, (1) Method, (b) Extraction of intracellular proteins.

### (c) Production of samples and protein chips for protein expression analysis, and expression analysis of intracellular proteins

100 µL of a sample produced by adjusting a cell lysate to 0.5 mg/mL using a dilution/wash buffer at pH 4 (50 mM sodium acetate buffer) (hereinafter, pH 4 buffer), was applied to spots of a cation exchange chip array (CM10, Bio-Rad) that had been pretreated with the pH 4 buffer, and the chip array was incubated for 30 minutes for reaction. Subsequently, the chip array was washed three times with the pH 4 buffer, and was rinsed two times with Milli-Q water. After the chip array was dried in air, 1.0 µL of energy absorbing molecules (SPA (EAM: saturated solution of sinapinic acid in 50% ACN/0.5% TFA solution) was used for examination on the high molecular weight side, and CHCA (α-cyano-4-hydroxycinnamic acid) was used for examination on the low molecular weight side) were applied onto each spot in two divided portions of 0.5 µL each. After the spot surfaces dried up, an analysis of the protein chip array was carried out.

Furthermore, 100 µL of a sample produced by adjusting a cell lysate to 0.5 mg/mL using a dilusion/wash buffer at pH 8 (50 mM Tris-HCl buffer) (hereinafter, pH 8 buffer), was applied to spots of an anion exchange chip array (Q10, Bio-Rad) that had been pretreated with the pH 8 buffer, and the chip array was incubated for one hour for reaction. Subsequently, the chip array was washed three times with the pH 8 buffer, and was rinsed two times with MILLI-Q water. After the chip array was dried in air, 1.0 µL of energy absorbing molecules (EAM and CHCA were used) were applied onto each spot in two divided portions of 0.5 µL each. After the spot surfaces dried up, an analysis of the protein chip array was carried out.

A protein expression analysis was carried out by surface-enhanced laser desorption/ionization time-of-flight mass spectrometry (SELDI-TOF MS). Regarding the analytic instrument, a ProteinChip™ Reader (Model PCS4000 Personal Edition, Bio-Rad) was used.

When SPA was used as the matrix, the analysis was performed under the conditions of mass range: 10,000 to 50,000 Daltons, focus mass: 18,000 Daltons, energy: 4,000 nJ, and 265 shots in total per sample.

When CHCA was used as the matrix, the analysis was performed under the conditions of mass range: 2,000 to 20,000 Daltons, focus mass: 7,700 Daltons, energy: 1,500 nJ, and 265 shots in total per sample.

Extraction of peaks having a signal-to-noise ratio (S/N ratio) of 5 or higher and a protein expression comparative analysis were carried out using CiphergenExpress™ Data Manager 3.0.

### (d) Selection of candidate peaks

When the protein peaks of the oxaliplatin-high-sensitivity cell lines and the oxaliplatin-low-sensitivity cell lines were compared, two kinds of peaks having a difference in the amounts of expression with p < 0.001 were finally extracted (candidate proteins A and B) . Furthermore, the isoelectric points of the candidate proteins A and B were estimated from the variations in the amounts of expression caused by variation of pH.

### (2) Results

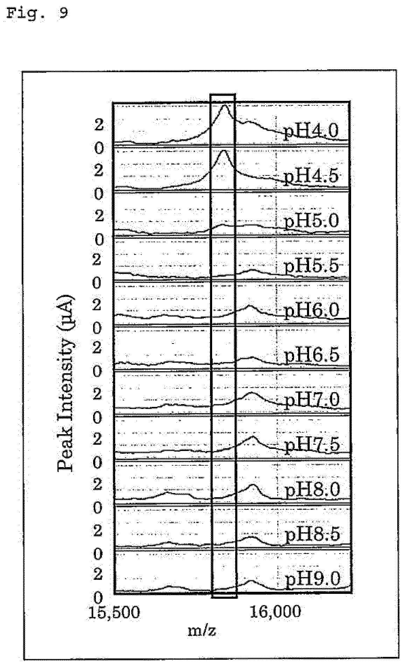

The candidate protein A found when CM10 was used as a chip, had an increased amount of expression in the oxaliplatin-high-sensitivity cell lines compared to the low-sensitivity cell lines, as shown inFig. 8. For the candidate protein A, the p value obtained by comparing the protein peaks of the oxaliplatin-high-sensitivity cell lines and the low-sensitivity cell lines was p = 7.9×10⁻⁶, and the candidate protein A had an m/z value of 15847Da and an isoelectric point (PI) of 4.5 to 5.5 (Fig. 9).

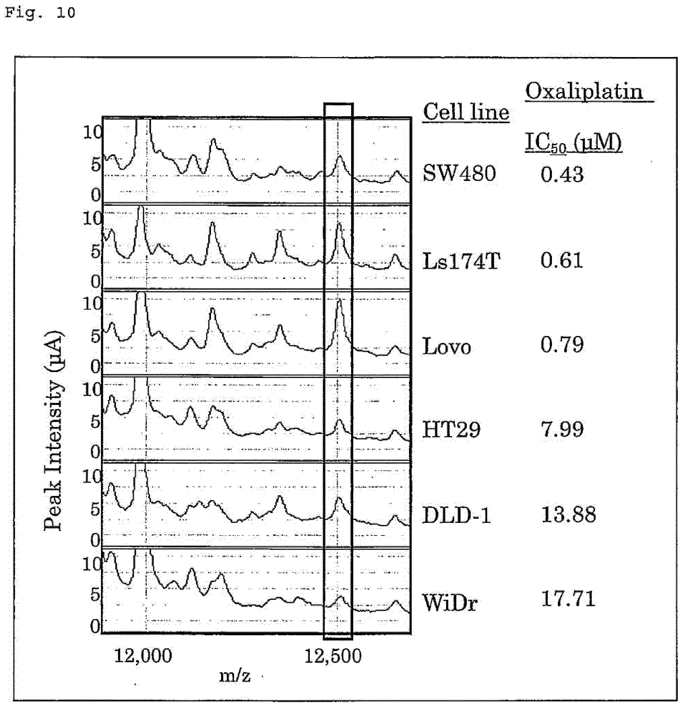

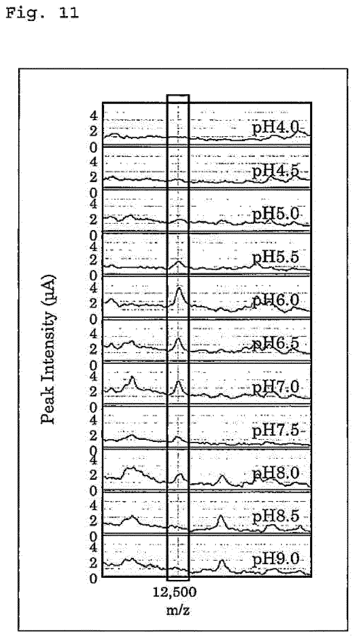

Furthermore, the candidate protein B found when Q10 was used as a chip, had an increased amount of expression in the oxaliplatin-high-sensitivity cell lines compared to the low-sensitivity cell lines, as shown in Fig. 10. For the candidate protein B, the p value obtained by comparing the protein peaks of the oxaliplatin-high-sensitivity cell lines and the low-sensitivity cell lines was p = 1.0×10⁻⁴, and the candidate protein B had an m/z value of 12506Da and an isoelectric point (PI) of 4.0 to 5.0 (Fig. 11).

As a result of the prediction of the candidate protein B obtained as described above based on the molecular weight and the isoelectric point obtained by the SELDI-TOF MS analysis by using a database (Swiss Prot: European Bioinformatics Institute), the candidate protein B was assumed to be cytochrome c oxygenase subunitVa (COX5A) (molecular weight: 12,501, isoelectric point: 4.88).

### Example 4

### Analysis of candidate protein A by two-dimensional electrophoresis

For the purpose of identifying the candidate protein A, the candidate protein A was further subjected to an analysis by two-dimensional electrophoresis.

Intracellular proteins were extracted by a method similar to that used in Example 2, section (1), Method (b) Extraction of intracellular proteins, using SW480, an oxaliplatin-high-sensitivity cell line, and WiDr, a low-sensitivity cell line. The extract was adjusted to a concentration of 5 mg/mL, and then stored at -80°C until use for analysis.

50 µg of the cell lysate was adjusted to 125 µL using a swelling solution (2 M thiourea, 7 Murea, 4% CHAPS, 2% Pharmalyte pH 3-10, and 1% Destreak Reagent) . The adjusted sample solution was centrifuged for 10 minutes at 4°C at 13,000×rpm, and the supernatant was added to Immobiline DryStrip gels (pH 3 to 10, non-linear, 13 cm, GE Healthcare Biosciences), and the gels were swollen. Subsequently, isoelectric focusing was performed. The conditions for the electrophoresis are presented in Table 7.

**[Table 7]**

| step | Voltage change pattern | Voltage (V) | Time (hr) | kVhr |
|---|---|---|---|---|
| 1 | Step and Hold | 300 | 0:30 | 0.2 |
| 2 | Gradient | 1000 | 0:30 | 0.3 |
| 3 | Gradient | 5000 | 1:20 | 4 |
| 4 | Step and Hold | 5000 | 0:50 | |
| 5 | Step and Hold | 500 | 2:00 | |

After completion of the isoelectric focusing, the Immobiline Drystrip gels were transferred into a 10 mL tube, the tube was filled with equilibration buffer A (50 mM Tris-HCl pH 8.8, 6 M Urea, 30% glycerol, 1% SDS, 0.25% DTT, and BPB), and the gels were equilibrated by shaking the tube for 15 minutes . The buffer was replaced with equilibration buffer B (50 mM Tris-HCl pH 8.8, 6MUrea, 30% glycerol, 1% SDS, 4.5% iodoacetamide, and BPB), and the gels were equilibrated by shaking the tube for 15 minutes. The Immobiline DryStrip gels that had been equilibrated were subjected to electrophoresis at a constant current of 20 mA. 10% to 20% polyacrylamide gradient gel (Biocraft) having a size of 16 × 16 cm was used as a gel.

Next, in order to collect the spots thus obtained, the gels were subjected to silver staining using a PlusOne Silver Staining Kit, Protein (GE Healthcare). Collection of the spots by silver staining was carried out by a method similar to the method used in Example 1, section (6) Collection of spots.

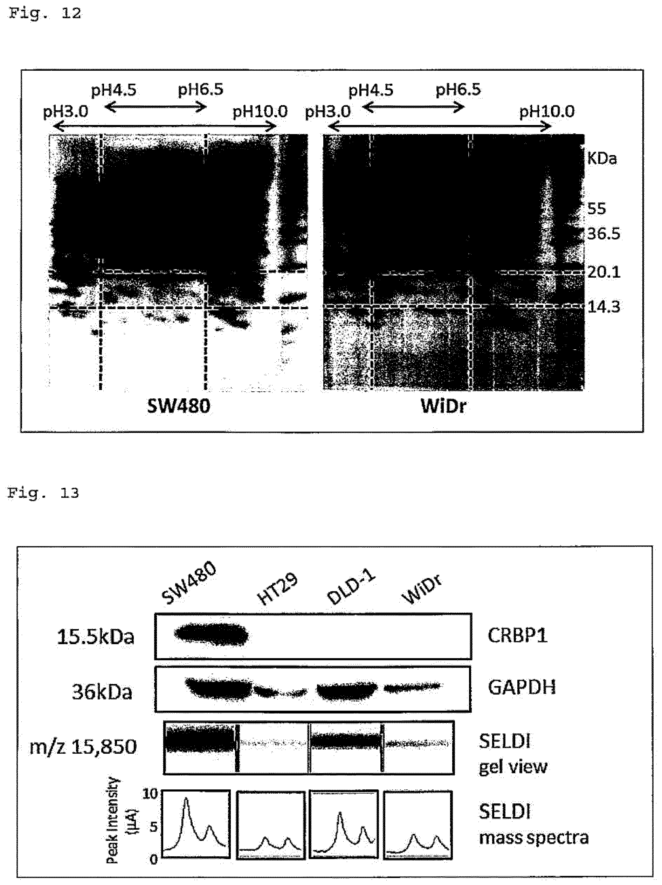

Based on the information (molecular weight and isoelectric point) on the candidate protein A obtained in Example 3, spots that had been isolated in the range of the molecular weight of 10,000 to 30,000 Da and the pH of approximately 4.5 to 6.5 (boxed part in Fig. 12) on the gels developed by two-dimensional electrophoresis, were designated as targets, and among these, eight spots that showed differences in the amounts of expression when a comparison was made between SW480 and WiDr, were selected and collected.

The spots thus collected were subjected to in-gel trypsin digestion of the proteins according to a known method, and then the spots were analyzed (MS/MS analysis) using LCMS-IT-TOF (liquid chromatograph mass spectrometer, Shimadzu). The results thus obtained were subjected to MASCOT database retrieval.

As a result of the database retrieval, cellular retinol binding protein1 (CRBP1) (molecular weight 15,850, isoelectric point 4.99) was identified. Since the molecular weight and the isoelectric point almost matched those of candidate protein A, CRBP1 was assumed to be the candidate protein A.

### Example 5

### Confirmation of protein expression by Western blotting (1) Confirmation of CRBP1

Intracellular proteins of the oxaliplatin-high-sensitivity cell lines and the low-sensitivity cell lines were extracted by a method similar to that used in Example 2, section (1), Method (b) Extraction of intracellular proteins, subsequently a protein sample was applied to 15% polyacrylamide gel in an amount of 50 µg per lane, and SDS-PAGE was performed at a constant current of 20 mA. After electrophoresis, proteins were blotted on a PVDF membrane using a dry blotting system (iBlot™, invitrogen), blockingwas carried out, and then a primary antibody reaction was performed using anti-CRBPlmonoclonal antibody (sc-53989, santa cruz) (×1/200), or using anti-GAPDH monoclonal antibody (Ambion) (x 1/10000) for endogenous proteins. The proteins were subjected to a secondary antibody reaction with alkali phosphatase-labeled anti-mouse IgG antibody (×1/20000), and then CDP-Star™chemiluminescent substrate was added thereto as a reaction substrate to cause luminescence. Detection was performed by means of a lumino image analyzer (LAS-4000 mini, Fujifilm). Regarding the blocking reagent, secondary antibody, and reaction substrate, a Chemiluminescent Western Blot Immunodetection Kit (WesternBreeze™, invitrogen) was used.

Expression of CRBP1 in the oxaliplatin-high-sensitivity cell lines was confirmed by Western blotting using an anti-CRBPl antibody (Fig. 13).

### (2) Confirmation of COX5A

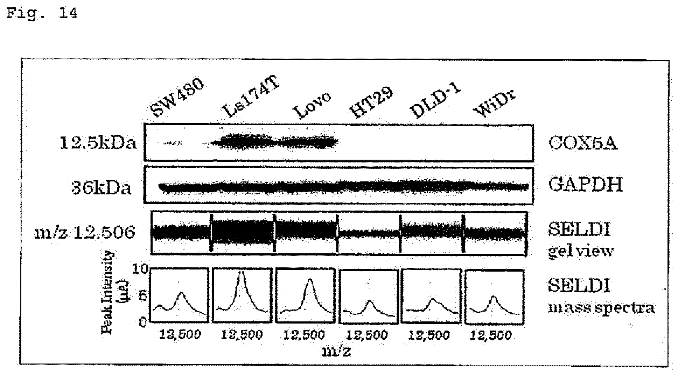

The confirmation was carried out by a method similar to that used in section (1) Confirmation of CRBP1, except that an anti-COX5Amonoclonal antibody (sc-376907, Santa Cruz) (×1/200 added) was used as the primary antibody. As a result, expression of COX5A in the oxaliplatin-high-sensitivity cell lines was confirmed by Western blotting using an anti-COX5A antibody (Fig. 14).

### Example 6

Change in sensitivity to oxaliplatin of human colorectal cancer cell lines caused by introduction of siRNA

### (1) Method

### (a) Cells used

Among the nine kinds of colorectal cancer cell lines described in Test Example 1, Ls174T was used as an oxaliplatin-high-sensitivity cell line, HCT116 was used as a moderate-sensitivity cell line, and HT29 and DLD-1 were used as low-sensitivity cell lines.

### (b) Drug

The oxaliplatin bulk powders described in Test Example 1 were used.

### (c) Introduction of siRNA into human colorectal cancer cell lines

Each of the human colorectal cancer cell lines was inoculated into a 6-well plate at a density of 1×10⁵ cells/well, and after 24 hours, the medium was replaced with serum-free DMEM medium (Wako, 044-29765). A solution obtained by dissolving 150 pmol per well of each of the siRNAs indicated in Table 8 in 250 µL of Opti MEM (GIBCO, No. 319985) was mixed with a solution obtained by mixing 4.5 µL of Lipofectamin RNAiMAX Reagent (Invitrogen, No. 13778-150) and 250 µL of Opti MEM (GIBCO, No. 319985). The resultant mixture was incubated for 10 to 20 minutes. After incubation, 500 µL each of the mixture was added to each well of the 6-well plate on which the human colorectal cancer cell lines were cultured, and the medium in each well was replaced with a serum-added DMEM medium (Wako, 044-29765) 4 to 6 hours after the addition. The cells were collected 24 hours after the addition of siRNA.

The combinations of the human colorectal cancer cell lines used and the siRNAs introduced thereinto are presented in Table 9. Regarding the control siRNA, siRNA of No. 4390843 of Life Technologies, Inc. was used.

**[Table 8]**

| siRNA name | Sequence (5'→>3') | SEQ ID NO |
|---|---|---|
| PHB siRNA | CGUGGGUACAGAAACCAAUtt (life technologies, s10424) | SEQ ID NO: 1 |
| ANXA5 siRNA | GUACAUGACUAUAUCAGGAtt (life technologies, s1392) | SEQ ID NO: 2 |
| TALDO siRNA | UGCUAUUGAUAAACUUUUUtt (life technologies, s13776) | SEQ ID NO: 3 |
| C1QBP siRNA | GGCCUUAUAUGACCACCUAtt (life technologies, s2139) | SEQ ID NO: 4 |
| IPYR siRNA | GGAAUCAGUUGCAUGAAUAtt (life technologies, s10878) | SEQ ID NO: 5 |

**[Table 9]**

| siRNA name | Name of human colorectal cancer cell line into which siRNA had been introduced |
|---|---|
| PHB siRNA | Ls174T, HCT116, HT29, DLD-1 |
| ANXA5 siRNA | HCT116, HT29 |
| TALDO siRNA | Ls174T, HCT116, HT29 |
| C1QBP siRNA | HCT116 |
| IPYR siRNA | Ls174T |

### (d) Method for measuring sensitivity to oxaliplatin of human colorectal cancer cell lines into which siRNA had been introduced

Measurement of the sensitivity to oxaliplatin (measurement of IC₅₀ values) of the human colorectal cancer cell lines into which siRNA had been introduced, was performed by the method described in Test Example 1(1)(c).

### (2) Results

### (a) PHB siRNA

The results are presented in Table 10. In each of the human colorectal cancer cell lines , Ls174T, HCT116, HT29 and DLD-1, in which PHB had been knocked out by introduction of siRNA, the IC₅₀ values increased, and the sensitivity to oxaliplatin decreased, compared to the case where the control siRNA had been introduced. These results coincided with the results of Example 2, in which the amount of expression of PHB in the oxaliplatin-high-sensitivity cell lines was large, while the amount of expression of PHB was small in the low-sensitivity cell lines.

**[Table 10]**

| Name of human colorectal cancer cell line into which siRNA had been introduced | siRNA name | IC₅₀ value (µM) | Significant difference (p) |
|---|---|---|---|
| Ls174T | ControlsiRNA | 0.86 | 0.033* |
| | PHB siRNA | 11.87 | |
| HCT116 | Control siRNA | 1.72 | 0.042* |
| | PHB siRNA | 21.48 | |
| HT29 | Control siRNA | 16.14 | 0.029* |
| | PHB siRNA | 41.90 | |
| DLD-1 | Control siRNA | 14.68 | 0.017* |
| | PHB siRNA | 52.34 | |

| | | | |
|---|---|---|---|
| *: p < 0.05 | | | |

### (b) ANXA5 siRNA

The results are presented in Table 11. In each of the human colorectal cancer cell lines, HCT116 and HT29, in which ANXA5 had been knocked out by introduction of siRNA, the IC₅₀ values decreased, and the sensitivity to oxaliplatin was enhanced, compared to the case where the control siRNA had been introduced. These results coincided with the results of Example 2, in which the amount of expression of ANXA5 in the oxaliplatin-high-sensitivity cell lines was small, while the amount of expression of ANXA5 in the low-sensitivity cell lines was large.

**[Table 11]**

| Name of human colorectal cancer cell line into which siRNA had been introduced | siRNA name | IC₅₀ value (µM) | Significant difference (p) |
|---|---|---|---|
| HCT116 | Control siRNA | 1.39 | 0.022* |
| | ANXA5 siRNA | 0.76 | |
| HT29 | Control siRNA | 16.14 | 0.027* |
| | ANXA5 siRNA | 5.35 | |

| | | | |
|---|---|---|---|
| *: p < 0.05 | | | |

### (c) TALDO siRNA

The results are presented in Table 12. In each of the human colorectal cancer cell lines, Ls174T, HCT116 and HT29, in which TALDO had been knocked out by introduction of siRNA, the IC₅₀ values decreased, and the sensitivity to oxaliplatin was enhanced, compared to the case where the control siRNA had been introduced. These results coincided with the results of Example 2, in which the amount of expression of TALDO in the oxaliplatin-high-sensitivity cell lines was small, while the amount of expression of TALDO in the low-sensitivity cell lines was large.

**[Table 12]**

| Name of human colorectal cancer cell line into which siRNA had been introduced | siRNA name | IC₅₀ value (µM) | Significant difference (p) |
|---|---|---|---|
| Ls174T | Control siRNA | 0.86 | 0.047* |
| | TALDO siRNA | 0.43 | |
| HCT116 | Control siRNA | 1.53 | 0.0081** |
| | TALDO siRNA | 0.73 | |
| HT29 | Control siRNA | 16.14 | 0.0022*** |
| | TALDO siRNA | 2.77 | |

| | | | |
|---|---|---|---|
| *: p < 0.05, **: p < 0.01, ***: p < 0.005 | | | |

### (d) C1QBP siRNA

The results are presented in Table 13. In HCT116 in which C1QBP had been knocked out by introduction of siRNA, the IC₅₀ value increased, and the sensitivity to oxaliplatin decreased, compared to the case where the control siRNA had been introduced. These results coincided with the results of Example 2, in which the amount of expression of C1QBP in the oxaliplatin-high-sensitivity cell lines was large, while the amount of expression of C1QBP was small in the low-sensitivity cell lines.

**[Table 13]**

| Name of human colorectal cancer cell line into which siRNA had been introduced | siRNA name | IC₅₀ value (µM) | Significant difference (p) |
|---|---|---|---|
| HCT116 | Control siRNA | 1.34 | 0.0060** |
| | C1QBP siRNA | 2.18 | |

| | | | |
|---|---|---|---|
| **: p < 0.01 | | | |

### (e) IPYR siRNA

The results are presented in Table 14. In Ls174T in which IPYR had been knocked out by introduction of siRNA, the IC₅₀ value decreased, and the sensitivity to oxaliplatin was enhanced, compared to the case where the control siRNA had been introduced. These results coincided with the results of Example 2, in which the amount of expression of IPYR in the oxaliplatin-high-sensitivity cell lines was small, while the amount of expression of IPYR was large in the low-sensitivity cell lines.

**[Table 14]**

| Name of human colorectal cancer cell line into which siRNA had been introduced | siRNA name | IC₅₀ value (µM) | Significant difference (p) |
|---|---|---|---|
| Ls174T | Control siRNA | 0.68 | 0.0056** |
| | IPYR siRNA | 0.39 | |

| | | | |
|---|---|---|---|
| **: p < 0.01 | | | |

## Claims

1. Use of TALDO as a marker for determining sensitivity to an anti-cancer agent, which anti-cancer agent is oxaliplatin or a salt thereof and which cancer is colorectal cancer.

2. A method for determining sensitivity to an anti-cancer agent, which anti-cancer agent is oxaliplatin or a salt thereof and which cancer is colorectal cancer, the method comprising a step of measuring an amount of TALDO in a biological sample derived from a cancer patient.

3. The determination method according to claim 2, further comprising a step of determining the sensitivity of the cancer patient to oxaliplatin or a salt thereof by comparing the measurement result with a control level.

4. The determination method according to claim 2 or 3, wherein the biological sample is a biological sample derived from a cancer patient to which oxaliplatin or a salt thereof has been administered.

5. Use of a kit for performing the determination method according to any one of claims 2 to 4, the kit comprising a protocol for measuring the amount of TALDO in a biological sample derived from a cancer patient.

6. A screening method for an anti-cancer agent sensitivity enhancer, wherein the anti-cancer agent is oxaliplatin or a salt thereof and which cancer is colorectal cancer, the method comprising employing, as an index, variation in expression of TALDO in a cancer cell line or a biological sample derived from a tumor-bearing animal in the presence of oxaliplatin or a salt thereof.

## Patentansprüche

1. Verwendung von Transaldolase (TALDO) als einen Marker zur Bestimmung des Ansprechverhaltens gegenüber einem Krebsmedikament, wobei das Krebsmedikament Oxaliplatin oder ein Salz davon und der Krebs ein Kolorektalkarzinom ist.

2. Ein Verfahren zur Bestimmung des Ansprechverhaltens gegenüber einem Krebsmedikament, wobei das Krebsmedikament Oxaliplatin oder ein Salz davon und der Krebs ein Kolorektalkarzinom ist, und das Verfahren einen Schritt zur Bestimmung einer Menge an TALDO in einer biologischen Probe umfasst, die einem Krebspatienten entnommen wurde.

3. Das Bestimmungsverfahren nach Anspruch 2, weiter umfassend einen Schritt zur Bestimmung des Ansprechverhaltens des Krebspatienten auf Oxaliplatin oder eines Salzes davon durch Vergleichen des Messergebnisses mit einem Referenzwert.

4. Das Bestimmungsverfahren nach Anspruch 2 oder 3, wobei die biologische Probe eine biologische Probe ist, die einem Krebspatienten entnommen wurde, dem Oxaliplatin oder ein Salz davon verabreicht wurde.

5. Die Verwendung eines Kits zur Durchführung des Bestimmungsverfahrens nach einem der Ansprüche 2 bis 4, wobei das Kit ein Protokoll zur Bestimmung der Menge an TALDO in einer biologischen Probe umfasst, die einem Krebspatienten entnommen wurde.

6. Ein Screening-Verfahren für ein Mittel, welches das Ansprechverhalten eines Krebsmedikaments verbessert, wobei das Krebsmedikament Oxaliplatin oder ein Salz davon und der Krebs ein Kolorektalkarzinom ist, und das Verfahren die Heranziehung der Variation der Expression von TALDO in einer Krebszelllinie oder einer biologischen Probe, welche von einem tumorausprägenden Tier in Gegenwart von Oxaliplatin oder einem Salz davon entnommen wurde, als Maßzahl umfasst.

## Revendications

1. Utilisation de transaldolase (TALDO) en tant que marqueur pour déterminer la sensibilité à un agent anticancéreux, lequel agent anticancéreux est l'oxaliplatine ou un sel de celui-ci et lequel cancer est un cancer colorectal.

2. Procédé pour déterminer la sensibilité à un agent anticancéreux, lequel agent anticancéreux est l'oxaliplatine ou un sel de celui-ci et lequel cancer est un cancer colorectal, le procédé comprenant une étape de mesure de la quantité de TALDO dans un échantillon biologique dérivé à partir d'un patient cancéreux.

3. Procédé de détermination selon la revendication 2, comprenant en outre une étape de détermination de la sensibilité du patient cancéreux à l'oxaliplatine ou à un sel de celui-ci par comparaison du résultat de mesure avec un niveau témoin.

4. Procédé de détermination selon la revendication 2 ou 3, dans lequel l'échantillon biologique est un échantillon biologique dérivé à partir d'un patient cancéreux auquel de l'oxaliplatine ou un sel de celui-ci a été administré.

5. Utilisation d'un kit pour mettre en oeuvre le procédé de détermination selon l'une quelconque des revendications 2 à 4, le kit comprenant un protocole pour mesurer la quantité de TALDO dans un échantillon biologique dérivé à partir d'un patient cancéreux.

6. Procédé de dépistage d'un amplificateur de sensibilité à un agent anticancéreux, dans lequel l'agent anticancéreux est l'oxaliplatine ou un sel de celui-ci, et lequel cancer est un cancer colorectal, le procédé comprenant l'emploi, en tant qu'indice, de la variation de l'expression de TALDO dans une lignée cellulaire cancéreuse ou un échantillon biologique dérivé à partir d'un animal portant une tumeur en présence d'oxaliplatine ou d'un sel de celui-ci.


### SEQUENCE LISTING

<110> KEIO UNIVERSITY KABUSHIKI KAISHA YAKULT HONSHA
<120> Marker for Determination of Sensitivity to Anticancer Agent
<130> YK0084
<150> JP 2014-171729
   <151> 2014-08-26
<160> 5
<170> PatentIn version 3.5
<210> 1
   <211> 21
   <212> DNA
   <213> Artificial Sequence
<220>
   <223> an artificially synthesized siRNA
<220>
   <221> misc_feature
   <222> (1)..(19)
   <223> RNA
<400> 1
   cguggguaca gaaaccaaut t 21
<210> 2
   <211> 21
   <212> DNA
   <213> Artificial Sequence
<220>
   <223> an artificially synthesized siRNA
<220>
   <221> misc_feature
   <222> (1)..(19)
   <223> RNA
<400> 2
   guacaugacu auaucaggat t 21
<210> 3
   <211> 21
   <212> DNA
   <213> Artificial Sequence
<220>
   <223> an artificially synthesized siRNA
<220>
   <221> misc_feature
   <222> (1)..(19)
   <223> RNA
<400> 3
   ugcuauugau aaacuuuuut t 21
<210> 4
   <211> 21
   <212> DNA
   <213> Artificial Sequence
<220>
   <223> an artificially synthesized siRNA
<220>
   <221> misc_feature
   <222> (1)..(19)
   <223> RNA
<400> 4
   ggccuuauau gaccaccuat t 21
<210> 5
   <211> 21
   <212> DNA
   <213> Artificial Sequence
<220>
   <223> an artificially synthesized siRNA
<220>
   <221> misc_feature
   <222> (1)..(19)
   <223> RNA
<400> 5
   ggaaucaguu gcaugaauat t 21